# EUROPÄISCHE PATENTSCHRIFT

(11) **EP 1 536 752 B1**
(45) Veröffentlichungstag und Bekanntmachung des Hinweises auf die Patenterteilung: **05.12.2007**
(21) Anmeldenummer: 03790800.1
(22) Anmeldetag: 16.07.2003
(51) Int. Cl.: A61K 8/04, A61K 8/92, A61K 8/37, A61K 8/39, A61K 8/55, A61Q 1/14, A61Q 19/10

(54) **Verfahren zur Herstellung einer kosmetischen und/oder dermatologischen Reinigungsemulsion**
Process for the preparation of a cosmetic and/or dermatological cleaning emulsion
Procédé de fabrication d'une émulsion nettoyante cosmétique et/ou dermatologique

(30) Priorität: 29.08.2002 DE 10239648
(43) Veröffentlichungstag der Anmeldung: 08.06.2005
(73) Patentinhaber: Beiersdorf AG, 20253 Hamburg (DE)
(72) Erfinder: NIELSEN, Jens, 24558 Henstedt-Ulzburg (DE); SCHÄFER, Andreas, 22299 Hamburg (DE); CAILLOUX, Birgit, 22339 Hamburg (DE); SEIBEL, Angelika, 22850 Norderstedt (DE)
(86) Internationale Anmeldenummer: PCT/EP2003/007729
(87) Internationale Veröffentlichungsnummer: WO 2004/019894

(56) Entgegenhaltungen:
- EP-A- 1 093 794
- EP-A- 1 352 639
- EP-A- 1 352 641
- WO-A-01/01949
- WO-A-95/17155
- DE-A- 10 063 659
- US-A- 5 725 844
- US-B1- 6 274 153

## Beschreibung

Die vorliegende Erfindung betrifft ein Verfahren zur Herstellung einer Kosmetischen und/oder dermatologischen Emulsion gemäß Anspruch 1.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte, in der Regel Cremes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement. Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 11 wasserlösliche, hydrophile.

Der HLB-Wert sagt etwas über das Gleichgewicht der Größe und Stärke der hydrophilen und der lipophilen Gruppen eines Emulgators aus. Aus diesen Überlegungen lässt sich ableiten, dass auch die Wirksamkeit eines Emulgators durch seinen HLB-Wert charakterisiert werden kann. Die folgende Aufstellung zeigt den Zusammenhang zwischen HLB-Wert und möglichem Anwendungsgebiet:

| **HLB-Wert** | **Anwendungsgebiet** |
|---|---|
| 0 bis 3 | Entschäumer |
| 3 bis 8 | W/O-Emulgator |
| 7 bis 9 | Netzmittel |
| 8 bis 18 | O/W-Emulgator |
| 12 bis 18 | Lösungsvermittler |

Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z.B. H.P.Fiedler., Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Ölphase entsteht unter sonst gleichen Herstellungsbedingungen eine W/O-Emulsion.

Die meisten Hautreinigungsprodukte werden in Form von wässrige Tensidzubereitungen angeboten. Bei Tensiden handelt es sich ebenfalls um oberflächenaktive Verbindungen, die in der Regel jedoch HLB-Werte aufweisen, die deutlich größer als 20 sind.

Wässrig-tensidische Reinigungszubereitungen haben häufig den Nachteil, die Haut leicht zu reizen (z.B. Schleimhautreizung an den Augen) und darüber hinaus sehr stark zu entfetten. Diese Effekte sind insbesondere bei empfindlicher Haut bzw. auf empfindlichen Hautpartien wie der Gesichtshaut (und hier besonders im Augenbereich) unerwünscht. Es gibt daher seit längerem Bemühungen Hautreinigungszubereitungen, insbesondere für die Gesichtshaut, auf Basis von Emulsionen zu entwickeln. Herkömmliche Gesichtsreinigungsemulsionen haben jedoch immer noch den Nachteil, dass sie entweder die Haut nicht gründlich genug reinigen und/oder einen unangenehmen sensorischen Eindruck auf der Haut hinterlassen. Es war daher die Aufgabe der vorliegenden Erfindung, diese Nachteile des Standes der Technik zu beseitigen und gründlich reinigende Reinigungszubereitungen zu entwickeln, welche die Haut nicht reizen, sondern vielmehr einen angenehmen samtig, weich, gepflegten und doch reinlichen sensorischen Eindruck auf der Haut zurückzulassen.

Ein weiterer Nachteil am Stande der Technik besteht in dem Umstand, das Emulsionen, beispielsweise im Vergleich zu wässrig-tensidischen Reinigungszubereitungen, besonders zeit- und energieaufwendig (und damit teuer) in der Herstellung sind. Üblicherweise werden Öl- und Wasserphase getrennt vorgelegt, auf 60 bis 90 °C erwärmt und anschließend voremulgiert (Hot/Hot-Prozess). Diese Voremulsion muss vor der Zugabe von Parfümöl und Farbstoffen wieder auf Raumtemperatur abgekühlt und anschließend nachemulgiert werden. In einigen Fällen kann von einer 20 bis 30 °C warmen Wasserphase ausgegangen werden. Bei diesem Hot/Cold-Prozess kann zum Teil auf das Abkühlen vor der Parfüm- und Farbstoffzugabe verzichtet werden. Nur in Ausnahmefällen ist es bisher jedoch möglich, nach dem besonders zeit- und energiesparenden sogenannten Cold/Cold-Prozess die Öl- und die Wasserphase bei Raumtemperatur miteinander zu emulgiem, da bei für dieses Verfahren eine bei Raumtemperatur dünnflüssige Ölphase vorliegen muss.

Es war daher eine weitere Aufgabe der vorliegenden Erfindung, die Emulsion möglichst zeit- und energiesparend herzustellen.

Überraschend gelöst werden die Aufgaben durch ein Verfahren zur Herstellung einer kosmetischen und/oder dermatologischen Emulsion enthaltend
a) eine wässrige Phase,
b) eine Ölphase, enthaltend mindestens 20 Gewichts-% mittelpolare Öle mit einer Polarität von 20 bis 35 mN/m, jeweils bezogen auf das Gesamtgewicht der Ölphase gewählt aus der Gruppe der Verbindungen Caprylylcarbonat, Isodecylneopentanoat, Isopropylpalmitat, Cyclomethicone,
   in einer Konzentration von 3 bis 15 Gewichts-%, jeweils bezogen auf das Gesamtgewicht der Emulsion
c) ein Emulgatorsystem aus
   i) einem oder mehreren Emulgatoren mit einem HLB-Wert von größer oder gleich 15 in einer Konzentration von 0,01 bis 2,5 Gewichts-%,
   ii) einem oder mehreren Emulgatoren mit einem HLB-Wert von kleiner oder gleich 10 in einer Konzentration von 0,05 bis 1,5 Gewichts-%,
jeweils bezogen auf das Gesamtgewicht der Emulsion,
neben gegebenenfalls weiteren kosmetischen Wirk-, Hilfs- und Zusatzstoffen, dadurch gekennzeichnet, dass die Ölphase bei einer Temperatur von 15 bis 50 °C mit dem Emulgatorsystem versetzt, in die 15 bis 25 °C warme wässrige Phase eingerührt und anschließend homogenisiert wird.

Die nach dem erfindungsgemäßen Verfahren hergestellten Emulsionen sind besonders gründlich in der Reinigung und dabei sanft und schonend zur Haut (insbesondere zur Gesichtshaut). Sie verleihen der Haut ein angenehm fettfreies, gepflegtes, samtig-weiches Hautgefühl, das auch längere Zeit anhält. Es tritt kein brennen beim Kontakt mit der Schleimhaut (z.B. am Auge) mehr auf. Darüber hinaus sind die erfindungsgemäßen Emulsionen besonders zeit- und energiesparend herstellbar, da sie alle nach dem Hot/Cold-Prozess hergestellt werden können. In der überwiegenden Zahl der Fälle können sie darüber hinaus erfindungsgemäß bevorzugt nach dem Cold/Cold-Prozess hergestellt werden.

Zwar kennt der Stand der Technik die Veröffentlichungen EP 1 352 639, EP 1 352 641, WO 95/17155, US 6274153, DE 100 63 659, EP 1 093 794 sowie WO 01/01949, doch konnten diese Schriften nicht den Weg zur vorliegenden Erfindung weisen.

Erfindungsgemäße Emulsionen bedeutet im Rahmen dieser Offenbarung Emulsionen, die nach dem erfindungsgemäßen Verfahren hergestellt werden.

Die erfindungsgemäßen Emulsionen sind erfindungsgemäß vorteilhaft frei von Tensiden.

Es ist erfindungsgemäß vorteilhaft, wenn der Anteil der wässigen Phase an der Emulsion von 50 bis 99 Gewichts-%, bevorzugt von 60 bis 95 Gewichts-% und ganz besonders bevorzugt von 75 bis 90 Gewichts-%, jeweils bezogen auf das Gesamtgewicht der Emulsion, beträgt.

Es ist dabei erfindungsgemäß vorteilhaft, wenn der Anteil der Ölphase an der Emulsion von 3 bis 15 Gewichts-%, jeweils bezogen auf das Gesamtgewicht der Emulsion, beträgt.

Dabei ist es erfindungsgemäß von Vorteil, wenn der Anteil an mittelpolaren Ölen mindestens 20 Gewichts-%, jeweils bezogen auf das Gesamtgewicht der Ölphase beträgt.

Bevorzugt im Sinne der vorliegenden Erfindung ist es, wenn die Polarität der mittelpolaren Öle 25 bis 35 mN/m beträgt.

Die Polarität kann dabei erfindungsgemäß bestimmt bzw. gemessen werden:

| | |
|---|---|
| Meßgerät: | Ringtensiometer (z.B. Krüss K 10) |
| Meßgröße: | spezifische Grenzflächenenergie = Grenzflächenspannung [Einheit: mN/m] |
| Untere Grenze: | 5 mN/m |

Geeignet für niedrigviskose Flüssigkeiten
Voraussetzung: Ausbildung einer Grenzfläche (Flüssigkeiten nicht mischbar)
Dichte der Flüssigkeit mit der größeren Oberflächenspannung muss größer sein als die mit der kleineren Oberflächenspannung.

Hierbei wird die resultierende spezifische Kraft (Energie) bestimmt, die benötigt wird, wenn die sich an der Grenzfläche befindlichen Moleküle auf der einen Seite mit den eigenen Molekülen, auf der anderen Seite mit den Molekülen der zweiten Flüssigkeit wechselwirken. Bei Mischbarkeit der Flüssigkeiten sind die Wechselwirkungen der Flüssigkeitsmoleküle verschiedener Substanzen thermodynamisch günstiger als die gleichartiger Moleküle. Ist bei Nichtmischbarkeit die resultierende Grenzflächenenergie klein, bedeutet das, dass eine Mischung der Flüssigkeiten zwar thermodynamisch ungünstiger ist als eine Phasentrennung, die Affinität zur anderen Flüssigkeit jedoch größer ist als bei großen Grenzflächenspannungen.

Erfindungsgemäß vorteilhaft enthält die erfindungsgemäße Emulsion einen oder mehrere Emulgatoren mit einem HLB-Wert von größer oder gleich 15 in einer Konzentration von 0,01 bis 2,5 Gewichts-%, bevorzugt in einer Konzentration von 0,05 bis 1,5 Gewichts-% und besonders bevorzugt in einer Konzentration von 0,1 bis 1 Gewichts-%, jeweils bezogen auf das Gesamtgewicht der Emulsion.

Erfindungsgemäß vorteilhaft enthält die erfindungsgemäße Emulsion einen oder mehrere Emulgatoren mit einem HLB-Wert von kleiner oder gleich 10 in einer Konzentration von 0,05 bis 1,5 Gewichts-% und besonders bevorzugt in einer Konzentration von 0,1 bis 1 Gewichts-%, jeweils bezogen auf das Gesamtgewicht der Emulsion.

Die erfindungsgemäße kosmetische und/oder dermatologische Emulsion kann vorteilhaft in Form einer O/W-, W/O/W-, O/W/O-, S/W-, S/W/S-, W/S/W -Emulsion vorliegen.

Als Silikonölemulsionen (W/S-, S/W-, etc) werden dabei erfindungsgemäß Emulsionen verstanden, die einen Silikonölanteil von 50 Gewichts-% oder großer 50 Gewichts-% in der lipophilen Phase, aufweisen.

Dabei ist es erfindungsgemäß bevorzugt, wenn es sich bei der Emulsion um eine O/W-Emulsion handelt.

Die erfindungsgemäße Emulsion kann als wässrige Phase neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol und Glycerin.

Als Öle mit einer Polarität von 20 bis 35 mN/m können erfindungsgemäß beispielsweise eingesetzt werden: Caprylylcarbonat (z.B. Cetiol®CC der Firma Cognis), Isodecylneopentanoat (z.B. DUB VCI 10 der Firma Stearinerie Dubois), Isopropylpalmitat (z.B., Estol 1515 der Firma Uniquema), Cyclomethicone (z.B. DC 345 der Firma Dow Corning.

Darüber hinaus kann die erfindungsgemäße Ölphase vorteilhaft weitere lipophile Inhaltsstoffe enthalten. So können diese unter anderem aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen gewählt werden. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether *(Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Corapan®TQ von Haarmann & Reimer*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 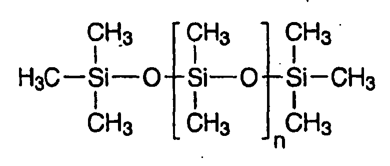 werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Als erfindungsgemäße Emulgatoren mit einem HLB-Wert von größer oder gleich 15 können beispielsweise die folgenden Verbindungen eingesetzt werden:
Polyethylenglycolether (z.B. Ceteareth-20, Eumulgin B 2),
Polyethylenglycolester (z.B. PEG-40 Stearate, Myric 52)
Sorbitanderivate (z.B. Polysorbate 80, Tween 80)
Zuckerester / -ether (z.B. Lauryl Glucoside, Plantacare 1200)
Phosphorsäureester / -salze: (z.B. Trilaureth-4 Phosphate, Hostaphat KL 340 N)

Als Emulgatoren mit einem HLB-Wert von kleiner oder gleich 10 können beispielsweise die folgenden Emulgatoren eingesetzt werden:
Polyethylenglycolether (z.B. Steareth-2, Brij 52)
Polyoxyethylen/Polydodecylglycol Blockpolymer: (z.B. PEG-22/Dodecyl Glycol Copolymer, Elfasos ST 9)
Glycerylester + Derivate: (z.B. Polyglyceryl-2 Dipolyhydroxystearate, Dehymuls PGPH)
Silikone: (z.B. Cyclopentasiloxane + PEG/PPG-18/18 Dimethicone, Abil EM 90)

Darüber hinaus kann die erfindungsgemäße Emulsion eine Vielzahl an kosmetischen und/oder dermatologischen Wirk-, Hilfs- und Zusatzstoffen enthalten.

Die erfindungsgemäßen Emulsionen können vorteilhaft anfeuchtende bzw. feuchthaltende Mittel (sogenannte Moisturizer) enthalten. Vorteilhafte feuchthaltende Mittel im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Emulsionen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in der Emulsion beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Emulsion.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Emulsion, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Emulsion, zu wählen.

Es ist insbesondere vorteilhaft, wenn die kosmetischen Emulsionen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin.

In die erfindungsgemäßen Emulsionen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin sowie Pflanzenwirkstoffe wie Azulen und Bisabolol, Glycyrrhizin, Hamamelin und Pflanzenextrakte wie Kamille, aloe vera, Hamazelis, Süßholzwurzel.

Die erfindungsgemäße Emulsion kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), Iodopropylbutylcarbamate (z.B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol.

Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Emulsion.

Erfindungsgemäß vorteilhaft, enthält die erfindungsgemäße Emulsion ein oder mehrere Hydrokolloide als Verdicker der Zubereitung. Der oder die erfindungsgemäßen Hydrokolloide können beispielsweise aus einer der folgenden Gruppen gewählt werden:
organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
organische, abgewandelte Naturstoffe, wie z. B. Carboxymethylcellulose und andere Celluloseether, Hydroxyethyl- und -propylcellulose und dergleichen,
organische, vollsynthetische Verbindungen, wie z. B. Polyacryl- und Polymethacryl-Verbindungen, Vinylpolymere, Polycarbonsäuren, Polyether, Polyimine, Polyamide,
anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose. Besonders bevorzugt ist die unter der Handelsbezeichnung Natrosot Plus 330 CS bei Aqualon erhältliche, auch als Cellulose Gum bezeichnete Natriumcarboxymethylcellulose.

Ein bevorzugtes Hydrokolloid im Sinne der vorliegenden Erfindung ist ferner Xanthan (CAS-Nr. 11138-66-2), auch Xanthan Gummi genannt.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Polyacrylate sind ebenfalls vorteilhaft im Sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Die Gesamtmenge an einem oder mehreren Hydrokolloiden wird in den fertigen kosmetischen oder dermatologischen Emulsionen vorteilhaft kleiner als 1,5 Gew.-%, bevorzugt zwischen 0,1 und 1,0 Gew.-%, bezogen auf das Gesamtgewicht der Emulsion, gewählt.

Es ist vorteilhaft im Sinn der vorliegenden Erfindung, wenn der Gehalt an einem oder mehreren Polyacrylaten in der kosmetischen oder dermatologischen Reinigungsemulsion aus dem Bereich von 0,1 bis 4 Gew.-%, ganz besonders vorteilhaft von 0,2 bis 2 Gew.-% gewählt wird, jeweils bezogen auf das Gesamtgewicht der Emulsion.

Die erfindungsgemäßen Emulsionen enthalten vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Die erfindungsgemäßen Zubereitungen können erfindungsgemäß vorteilhaft einen oder mehrere UV-Lichtschutzfiltersubstanzen enthalten. Dabei sind alle UV-Lichtschutzfilter erfindungsgemäß vorteilhaft einsetzbar, die nach der Kosmetikverordnung für die Verwendung in Kosmetika zugelassen sind. Es kann sich dabei erfindungsgemäß um wasserlösliche, öllösliche und oder pigmentäre Lichtschutzfilter handeln.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, weitere Pflanzenextrakte, Vitamine, Wirkstoffe, Konservierungsmittel, Bakterizide, Selbstbräuner, Depigmentierungsmittel, Pigmente, die eine färbende Wirkung haben, weichmachende, anfeuchtende und/oder feuchthaltende Substanzen, oder andere übliche Bestandteile einer kosmetischen oder dermatologischen Formulierung wie Polymere, Schaumstabilisatoren und Elektrolyte.

Es ist vorteilhaft im Sinne der vorliegenden Erfindung, wenn die Emulsion eine Viskosität von 10 bis 20000 mPas, bevorzugt von 50 bis 10000 mPas und ganz besonders bevorzugt von 100 bis mPas aufweist.

Erfiridungsgemäß vorteilhaft kann die erfindungsgemäße Emulsion auf ein Substrat aufgetragen sein. Die erfindungsgemäßen Substrate können glatt oder auch oberflächenstrukturiert sein. Erfindungsgemäß bevorzugt sind oberflächenstrukturierte Substrate.

Bei den erfindungsgemäßen Substraten kann die Gewebebildung durch Kette und Schuss, durch Maschenbildung oder durch Verschlingung, und/oder kohäsive und/oder adhäsive Verbindung von Textilfasern erfolgen. Dabei ist es erfindungsgemäß bevorzugt, wenn es sich bei dem Substrat um ein Verbundstoff handelt.

Erfindungsgemäß bevorzugt werden Substrate in Form von Tüchern eingesetzt, welche aus Vlies bestehen, insbesondere aus wasserstrahlverfestigten und/oder wasserstrahlgeprägten Vlies. Die Substrate können vorteilhaft auch als Bausch, gelochtes Vlies oder Netz ausgeführt sein.

Derartige Substrate können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Es hat sich als vorteilhaft herausgestellt für das Tuch, wenn dieses ein Gewicht von 20 bis 120 g/m², vorzugsweise von 30 bis 80 g/m² besonders bevorzugt 40 bis 60 g/m² hat (gemessen bei 20 °C ± 2 °C und bei einer Feuchtigkeit der Raumluft von 65 % ± 5 % für 24 Stunden).

Die Dicke des Substrates beträgt vorzugsweise 0,2 mm bis 2 mm, insbesondere 0,4 mm bis 1,5 mm, ganz besonders bevorzugt 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien-Viskose;-Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstofffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer vorteilhaften Ausführungsform des Vlieses bestehen die Fasern aus einer Mischung aus 60 % bis 80 %Viskose mit 40% bis 20 % PET, insbesondere 70% Viskose und 30 % PET. Besonders vorteilhaft ist eine Mischung aus 70 %Viskose und 30 % PET.

Erfindungsgemäß vorteilhaft kann ein erfindungsgemäßes Vlies ein Gemisch aus drei verschiedenen Fasermaterialien aufweisen. In einem solchen Falle ist eine Mischung aus 40 % bis 80 %Viskose mit 50% bis 20 % PET und 1 bis 30% Baumwolle bevorzugt. Erfindungsgemäß besonders bevorzugt ist eine Mischung aus 40 %Viskose und 50 % PET und 10 % Baumwolle.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Ferner weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Vorteilhafte Tücher im Sinne der vorliegenden Erfindung haben eine Reißkraft von insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Tuches beträgt vorzugsweise

| | | |
|---|---|---|
| im trockenen Zustand | Maschinenrichtung | 15 % bis 100 %, bevorzugt 20 % und 50 % |
| | Querrichtung | 40 % bis 120 %, bevorzugt 50 % und 85% |
| im getränkten Zustand | Maschinenrichtung | 15 % bis 100 %, bevorzugt 20 % und 40 % |
| | Querrichtung | 40 % bis 120 %, bevorzugt 50 % und 85 % |

In einer erfindungsgemäß besonderen Darreichungsform derartiger erfindungsgemäßer Substrate, kann das Substrat nach der Imprägnierung mit der Emulsion getrocknet werden um anschließend dem Anwender in Form eines trockenen Reinigungstuches dargereicht zu werden.

Eine andere erfindungsgemäß vorteilhafte Ausführungsform der vorliegende Erfindung stellen erfindungsgemäße Emulsionen dar, welche in Form eines Sprays dargereicht werden. Dieses kann in Form eines Pumpsprays oder mit einem Treibgas versehen in einer Aerosoldose vorliegen.

Das Treibgas wird erfindungsgemäß in einer Menge von 0,5 bis 20 Gewichts-%, besonders vorteilhaft in einer Menge von 5 bis 15 und ganz besonders vorteilhaft in einer Menge von 8 bis 11 Gewichts-%, jeweils bezogen auf das Gesamtgewicht der Emulsion eingesetzt. Erfindungsgemäß besonders vorteilhafte Treibgase sind Propan, Isobutan und n-Butan sowie deren Mischungen.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die erfindungsgemäße Emulsion liegt erfindungsgemäß bevorzugt in Form einer Reinigungsmilch oder Reinigungslotion vor. Diese kann vorteilhaft in einer Flasche oder Quetschflasche aufbewahrt und aus dieser heraus angewendet werden. Entsprechend sind auch Flaschen oder Quetschflaschen, welche eine erfindungsgemäße Zubereitung enthalten, erfindungsgemäß.

Erfindungsgemäß ist insbesondere das Verfahren zur Herstellung einer erfindungsgemäßen Emulsion welches dadurch gekennzeichnet ist, dass die Ölphase bei einer Temperatur von 15 bis 50 °C mit dem Emulgatorsystem versetzt, in die 15 bis 25 °C warme wässrige Phase eingerührt und anschließend homogenisiert wird. Anschließend kann Emulsion bei Raumtemperatur mit Farbstoff und/oder Parfüm versetzt und abermals homogenisiert werden, wobei die Hochdruckhomogenisation erfindungsgemäß besonders bevorzugt ist.

Erfindungsgemäß besonders bevorzugt ist es dabei, wenn sowohl die wässrige Phase als auch die Ölphase bei Raumtemperatur in flüssiger Form vorliegen, da in diesem Falle beide Phasen bei Raumtemperatur (in der Regel 15-25 °C, bevorzugt 20 °C) miteinander vermischt und anschließend homogenisiert werden können, wodurch sich die erfindungsgemäße Zeit- und Energleersparnis des Herstellungsverfahrens weiter erhöht.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Öl-in-Wasser- Reinigungsemulsionen**

| | **1** | **2** | **3** | **4** | **5** |
|---|---|---|---|---|---|
| Laurylglucosid | 1,0 | 0,1 | 1,5 | 0,6 | 0,8 |
| Diglycerindipolyhydroxystearat | 0,2 | 1,6 | 0,5 | 1,0 | 0,7 |
| Isopropylpalmitat | 2,0 | 8,0 | --- | --- | --- |
| Capryrylcarbonat | --- | 1,0 | 6,0 | 2,5 | --- |
| Cyclomethicon | 1,0 | 2,0 | --- | --- | --- |
| Isodecylneopentanoat | 7,5 | --- | --- | 12,0 | 8,0 |
| Cetylstearylalkohol | 0,1 | --- | 2,0 | --- | 1,0 |
| hydriertes Polyisobuten | --- | 1,0 | 2,5 | 0,5 | --- |
| Octyldodecanol | 5,0 | --- | --- | 2,5 | --- |
| 2-Methyl-1,3-propandiol | 5,0 | 2,5 | 1,0 | 8,5 | 0,1 |
| Panthenol | 0,5 | 1,0 | 0,75 | --- | --- |
| Glycerin | 3,0 | 5,0 | --- | 2,0 | --- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

## Patentansprüche

1. Verfahren zur Herstellung einer kosmetischen und/oder dermatologischen Emulsion enthaltend
a) eine wässrige Phase,
b) eine Ölphase, enthaltend mindestens 20 Gewichts-% mittelpolare Öle mit einer Polarität von 20 bis 35 mN/m, jeweils bezogen auf das Gesamtgewicht der Ölphase gewählt aus der Gruppe der Verbindungen Caprylylcarbonat, Isodecylneopentanoat, Isopropylpalmitat, Cyclomethicone,
in einer Konzentration von 3 bis 15 Gewichts-%, jeweils bezogen auf das Gesamtgewicht der Emulsion
c) ein Emulgatorsystem aus
i) einem oder mehreren Emulgatoren mit einem HLB-Wert von größer oder gleich 15 in einer Konzentration von 0,01 bis 2,5 Gewichts-%, jeweils bezogen auf das Gesamtgewicht der Emulsion
ii) einem oder mehreren Emulgatoren mit einem HLB-Wert von kleiner oder gleich 10 in einer Konzentration von 0,05 bis 1,5 Gewichts-%,
jeweils bezogen auf das Gesamtgewicht der Emulsion,
neben gegebenenfalls weiteren kosmetischen Wirk-, Hilfs- und Zusatzstoffen, **dadurch gekennzeichnet, dass** die Ölphase bei einer Temperatur von 15 bis 50 °C mit dem Emulgatorsystem versetzt, in die 15 bis 25 °C warme wässrige Phase eingerührt und anschließend homogenisiert wird.

2. Verfahren nach Anspruch 1, **dadurch gekennzeichnet, dass** es sich um eine O/W-, W/O/W- oder O/W/O-Emulsion handelt.

3. Verfahren nach einem der vorhergehenden Ansprüche, **dadurch gekennzeichnet, dass** es sich um eine O/W-Emulsion handelt.

4. Verfahren nach einem der vorhergehenden Ansprüche, **dadurch gekennzeichnet, dass** als Emulgatoren mit einem HLB-Wert von größer oder gleich 15 Polyethylenglycolester, Sorbitanderivate, Zuckerester / -ether und/oder Phosphorsäureester / -salze eingesetzt werden.

5. Verfahren nach einem der vorhergehenden Ansprüche, **dadurch gekennzeichnet, dass** als Emulgatoren mit einem HLB-Wert von kleiner oder gleich 10 Polyethylenglycolether, Polyoxyethylene/Polydodecylglycol Blockpolymere, Glycerylester + Derivate und/oder Silikone eingesetzt werden.

## Claims

1. Process for the preparation of a cosmetic and/or dermatological emulsion comprising
a) an aqueous phase,
b) an oil phase comprising at least 20% by weight of medium-polarity oils with a polarity of from 20 to 35 mN/m, in each case based on the total weight of the oil phase, selected from the group of the compounds caprylyl carbonate, isodecyl neopentanoate, isopropyl palmitate, cyclomethicones
in a concentration of from 3 to 15% by weight, in each case based on the total weight of the emulsion,
c) an emulsifier system of
i) one or more emulsifiers with an HLB value of greater than or equal to 15 in a concentration of from 0.01 to 2.5% by weight, in each case based on the total weight of the emulsion,
ii) one or more emulsifiers with an HLB value of less than or equal to 10 in a concentration of from 0.05 to 1.5% by weight,
in each case based on the total weight of the emulsion,
besides optionally further cosmetic active ingredients, auxiliaries and additives, **characterized in that** the oil phase is admixed at a temperature of from 15 to 50°C with the emulsifier system, stirred into the 15 to 25°C-hot aqueous phase and then homogenized.

2. Process according to Claim 1, **characterized in that** it is an O/W, W/O/W or O/W/O emulsion.

3. Process according to one of the preceding claims, **characterized in that** it is an O/W emulsion.

4. Process according to one of the preceding claims, **characterized in that** the emulsifiers with an HLB value of greater than or equal to 15 used are polyethylene glycol esters, sorbitan derivatives, sugar esters/ethers and/or phosphoric acid esters/salts.

5. Process according to one of the preceding claims, **characterized in that** the emulsifiers with an HLB value of less than or equal to 10 used are polyethylene glycol ethers, polyoxyethylene/polydodecyl glycol block polymers, glyceryl esters + derivatives and/or silicones.

## Revendications

1. Procédé de préparation d'une émulsion cosmétique et/ou dermatologique comprenant :
a) une phase aqueuse,
b) une phase huileuse comprenant au moins 20 % en poids d'huiles moyennement polaires présentant une polarité de 20 à 35 mN/m, dans chaque cas par rapport au poids total de la phase huileuse, choisies dans le groupe constitué des composés,
carbonate de caprylyle, néopentanoate d'isodécyle, palmitate d'isopropyle, cyclométhicones,
en une concentration de 3 à 15 % en poids, dans chaque cas par rapport au poids total de l'émulsion,
c) un système émulsifiant à base :
i) d'un ou de plusieurs agents émulsifiants présentant une valeur de HLB supérieure ou égale à 15 en une concentration de 0,01 à 2,5 % en poids, dans chaque cas par rapport au poids total de l'émulsion,
ii) d'un ou de plusieurs agents émulsifiants présentant une valeur de HLB inférieure ou égale à 10 en une concentration de 0,05 à 1,5 % en poids, dans chaque cas par rapport au poids total de l'émulsion,
éventuellement, en plus, d'ingrédients actifs, d'auxiliaires et d'additifs cosmétiques supplémentaires, **caractérisés en ce que** la phase huileuse est mélangée avec un système émulsifiant à une température de 15 à 50°C, agitée dans la phase aqueuse tiède à 15-25°C et ensuite homogénéisée.

2. Procédé selon la revendication 1, **caractérisé en ce qu'**il s'agit d'une émulsion H/E, E/H/E ou H/E/H.

3. Procédé selon l'une quelconque des revendications précédentes, **caractérisé en ce qu'**il s'agit d'une émulsion H/E.

4. Procédé selon l'une quelconque des revendications précédentes, **caractérisé en ce que**, en tant qu'agents émulsifiants présentant une valeur de HLB supérieure ou égale à 15, on utilise des esters de polyéthylèneglycol, des dérivés de sorbitane, des esters/éthers glucidiques et/ou des esters/sels de l'acide phosphorique.

5. Procédé selon l'une quelconque des revendications précédentes, **caractérisé en ce que**, en tant qu'agents émulsifiants présentant une valeur de HLB inférieure ou égale à 10, on utilise des éthers de polyéthylèneglycol, des copolymères séquencés polyoxyéthylène/polydodécylglycol, des esters glycéryliques + des dérivés et/ou des silicones.
